# EUROPÄISCHE PATENTSCHRIFT

(11) **EP 3 077 356 B1**
(45) Veröffentlichungstag und Bekanntmachung des Hinweises auf die Patenterteilung: **17.01.2018**
(21) Anmeldenummer: 14799348.9
(22) Anmeldetag: 14.11.2014
(51) Int. Cl.: C07C 45/50, C07C 45/62, C07C 45/66, C07C 45/75, C07C 51/235, C07C 47/02, C07C 47/19, C07C 49/21, C07C 53/126

(54) **VERFAHREN ZUR HERSTELLUNG VON ISOMEREN HEXANSÄUREN AUS DEN BEI DER HERSTELLUNG VON PENTANALEN ANFALLENDEN NEBENSTRÖMEN**
METHOD FOR PRODUCING ISOMERIC HEXANOIC ACIDS FROM THE SUBSIDIARY FLOWS ARISING DURING THE PRODUCTION OF PENTANALS
PROCÉDÉ DE PRÉPARATION D'ACIDES CAPROÏQUES ISOMÈRES À PARTIR DES FLUX SECONDAIRES PRODUITS LORS DE LA PRÉPARATION DE PENTANALS

(30) Priorität: 05.12.2013 DE 102013020323
(43) Veröffentlichungstag der Anmeldung: 12.10.2016
(73) Patentinhaber: OXEA GmbH, 46147 Oberhausen (DE)
(72) Erfinder: THEUERKAUF, Jens, 47802 Krefeld (DE); STRUTZ, Heinz, 47445 Moers (DE)
(86) Internationale Anmeldenummer: PCT/EP2014/003055
(87) Internationale Veröffentlichungsnummer: WO 2015/082042

(56) Entgegenhaltungen:
- EP-A1- 1 854 778
- WO-A2-2009/024549
- DE-A1- 3 744 212
- US-A- 2 115 892

## Beschreibung

Die vorliegende Erfindung betrifft ein Verfahren zur Herstellung von isomeren Hexansäuren aus Nebenströmen, die bei der Herstellung von Pentanalen anfallen.

Pentanale, auch Valeraldehyde genannt, haben als Zwischenprodukte in der industriellen organischen Chemie wirtschaftliche Bedeutung erlangt. Sie kommen in vier verschiedenen Strukturisomeren als lineares n-Pentanal, verzweigtes 2-Methylbutanal, verzweigtes 3-Methylbutanal sowie als hochverzweigtes 2,2-Dimethylpropanal oder Pivalaldehyd vor. Pentanale können als solche verwendet werden, beispielsweise für die Herstellung von Riechstoffen oder in Form ihrer Derivatisierungsprodukte, wie Pentanole, Pentansäuren oder Pentylamine. Dabei können die Pentanale in reiner Isomerenform oder als Isomerengemisch verarbeitet werden (Weissermel, Arpe, Industrielle Organische Chemie, 3. Auflage, VCH Verlagsgesellschaft mbH, Weinheim, 1988, Seite 218; Schneidmeir, Chemiker-Zeitung, 96. Jahrgang (1972), Nr. 7, Seiten 383-387).

Aufgrund der reaktiven Aldehydgruppe können Pentanale im basischen Medium eine Aldoladditionsreaktion zu Pentanaldimerisierungsprodukten mit zehn Kohlenstoffatomen eingehen. Besitzt mindestens eines der Pentanalisomere zwei reaktive, zur Carbonylgruppe α-ständige Wasserstoffatome, kann das zunächst gebildete Aldoladditionsprodukt unter Wasserabspaltung in das α,β-ungesättigte Decenal übergehen. Beispielsweise bildet sich aus der Selbstaddition von n-Pentanal mit nachfolgender Wasserabspaltung 2-Propylheptenal, das durch Kompletthydrierung in 2-Propylheptanol überführt werden kann, das als Weichmacheralkohol Verwendung findet (EP 0 366 089 A2). Die Selektivhydrierung von 2-Propylheptenal ergibt zunächst 2-Propylheptanal, das durch anschließende Oxidation in 2-Propylheptansäure überführt werden kann. 2-Propylheptansäure kann anschließend als Säurekomponente zur Herstellung von Schmiermittelestern verwendet werden.

Pentanale werden technisch durch Umsetzung von Butenen mit Synthesegas, einem Gemisch aus Kohlenmonoxid und Wasserstoff, in Gegenwart von Übergangsmetallverbindungen hergestellt. Man bezeichnet die Reaktion von Olefinen mit Synthesegas auch als Hydroformylierungsreaktion oder Oxo-Reaktion und man erhält bei der Hydroformylierung von Buten-1 neben dem geradkettigen n-Aldehyd n-Pentanal auch gewisse Anteile an dem iso-Aldehyd 2-Methylbutanal (Ullmann's Encyclopedia of Industrial Chemistry, 6th. Edition, 2003, Wiley-VCH Verlag GmbH & Co. KGaA, Weinheim, Vol. 2, Seiten 73-74; Vol. 25, Seiten 286-289).

Butene werden technisch durch die Dampfspaltung von Naphtha gewonnen. Üblicherweise wird aus dem Butenschnitt der Naphthaspaltung zunächst 1,3-Butadien unter Bildung von Raffinat I und anschließend Isobuten unter Bildung von Raffinat II abgetrennt (Weissermel, Arpe, Industrielle Organische Chemie, 3. Auflage, VCH Verlagsgesellschaft mbH, 1988, Seiten 71-79). Für die nachfolgende Hydroformylierungsreaktion setzt man überwiegend Raffinat II ein, in dem ein geringer Isobutenrestanteil zugelassen werden kann. In Sonderfällen kann auch Raffinat I mit einem hohen Isobutenanteil verarbeitet werden, gelegentlich auch ein an Buten-1 abgereichertes Raffinat II, das man auch als Raffinat III bezeichnet.
Die Hydroformylierungsreaktion kann sowohl in Anwesenheit als auch in Abwesenheit von komplexbildenden Verbindungen, beispielsweise in Gegenwart von Organophosphorverbindungen, durchgeführt werden. Nach EP 0 366 089 A2 arbeitet man in einer homogenen organischen Lösung unter Katalyse von Rhodium-Triphenylphosphin. Da im Allgemeinen ein möglichst hoher Anteil an n-Pentanal gegenüber 2-Methybutanal im gebildeten Pentanalgemisch angestrebt wird, wird die Hydroformylierungsreaktion häufig in Gegenwart von homogen gelösten Übergangsmetallkomplexen durchgeführt, die zunächst eine Isomerisierung des Butens-2 in Buten-1 ermöglichen, das dann im überwiegenden Maße zu n-Pentanal hydroformyliert wird. Für die isomerisierende Hydroformylierung eines Gemisches linearer Butene geeignete Rhodiumkomplexkatalysatoren werden beispielsweise in DE 102 25 282 A1 beschrieben, in denen die Komplexliganden ein Xanthengerüst besitzen.

Rhodiumkomplexkatalysatoren auf Basis von Bisphosphit-Liganden zusammen mit sterisch gehinderten sekundären Aminen, die ebenfalls für die isomerisierende Hydroformylierung eines Gemisches linearer Butene geeignet sind, werden in DE 10 2008 002 187 A1 behandelt. Auch zweistufige Verfahrensvarianten sind bekannt, beispielsweise nach DE 43 33 324 A1, DE 42 10 026 A1, DE 101 08 474 A1 und DE 101 08 475. In der ersten Stufe wird vorzugsweise Buten-1 umgesetzt während in der zweiten Stufe das Buten-2-haltige Abgas aus der ersten Stufe zu einem Gemisch aus n-Pentanal und 2-Methylbutanal hydroformyliert wird. Nach DE 43 33 324 A1 und DE 101 08 474 A1 kann die erste Hydroformylierungsstufe auch in Gegenwart wasserlöslicher Rhodiumkomplexkatalysatoren durchgeführt werden. Bei dieser Art der Reaktionsführung liegt eine flüssige, wässrige Katalysatorlösung neben der flüssigen, organischen Reaktionslösung vor, die nach Verlassen der Hydroformylierungszone auf einfache Weise voneinander durch Phasentrennung separiert werden können. Aufgrund des Vorliegens einer wässrigen und einer organischen flüssigen Phase bezeichnet man diese Art der Reaktionsführung auch als heterogenes Verfahren oder Zweiphasenverfahren.

Je nach der Zusammensetzung des Buteneinsatzgemisches und den Reaktionsbedingungen in der Hydroformylierungsstufe erhält man ein Pentanalgemisch mit wechselnden Anteilen an n-Pentanal, 2-Methylbutanal, 3-Methyl-butanal und geringen Menge an Pivalaldehyd, das üblicherweise aufdestilliert wird. N-Pentanal besitzt bei Normaldruck einen Siedepunkt von 103°C und kann mit dem Höhersiederstrom abgetrennt werden. Aufgrund der geringen Siedepunktsdifferenz zwischen 2-Methylbutanal (92°C bei Normaldruck) und 3-Methylbutanal (92,5°C bei Normaldruck) lässt sich 3-Methylbutanal bei vertretbarem Destillationsaufwand nicht vollständig vom 2-Methylbutanal abtrennen. Wenn man möglichst reines n-Pentanal gewinnen will, wird der Destillationsschritt häufig so gelegt, dass das flüchtigere 2-Methylbutanal neben dem Gehalt an 3-Methylbutanal noch erhebliche Mengen an n-Pentanal enthält, die mit den flüchtigen Bestandteilen abgeführt werden. Es besteht daher Bedarf an einem Verfahren, die im abgetrennten 2-Methyl-butanal vorliegenden Mengen an n-Pentanal und die vorliegenden Restgehalte an 3-Methylbutanal möglichst wirtschaftlich zu verwerten. Gleichzeitig soll 2-Methylbutanal mit einem Restgehalt an 3-Methylbutanal von weniger als 0,2 Gew.-%, bezogen auf den organischen Anteil, erhalten werden, das für besondere Anwendungen, beispielsweise für die Riechstoffherstellung nachgefragt wird.

Die vorliegende Erfindung besteht daher in einem Verfahren zur Herstellung von isomeren Hexansäuren enthaltend 2-Methylpentansäure aus den bei der Herstellung von Pentanalen anfallenden Nebenströmen. Es ist dadurch gekennzeichnet, dass man
a) ein Gemisch enthaltend lineare Butene in Gegenwart von Übergangsmetallverbindungen der Gruppe VIII des Periodensystems der Elemente mit Kohlenmonoxid und Wasserstoff bei erhöhter Temperatur und erhöhtem Druck zu einem Pentanalgemisch umsetzt;
b) das gemäß Schritt a) erhaltene Gemisch in einen mit 2-Methylbutanal und 3-Methylbutanal angereicherten Stoffstrom und in einen mit n-Pentanal angereicherten Stoffstrom trennt;
c) den gemäß Schritt b) mit 2-Methylbutanal und 3-Methylbutanal angereicherten Stoffstrom mit Formaldehyd umsetzt;
d) das nach Schritt c) erhaltene Reaktionsgemisch in Gegenwart eines Hydrierkatalysators mit Wasserstoff bei erhöhter Temperatur und erhöhtem Druck selektiv zu einem Gemisch enthaltend 2-Methyl-butanal und isomere Hexanale umsetzt;
e) das gemäß Schritt d) erhaltene Reaktionsgemisch in einen mit 2-Methylbutanal angereicherten Stoffstrom und in einen mit einem Gemisch isomerer Hexanale angereicherten Stoffstrom auftrennt; und
f) das gemäß Schritt e) erhaltene Gemisch isomerer Hexanale zu einem Gemisch isomerer Hexansäuren enthaltend 2-Methylpentansäure oxidiert.

Nach US 2 115 892 A und WO 2009/024549 A2 ist die Herstellung von einzelnen Hexansäuren aus entsprechenden Hexanalen wie 2-Ethylbutanal oder 2-Methylpentanal durch Oxidation mit Sauerstoff mit oder ohne Katalysator bekannt. Die Herkunft der zugrunde liegenden Aldehyde bleibt offen.

Einsatzstoffe für das erfindungsgemäße Verfahren sind Kohlenwasserstoffgemische, die typischerweise keine oder sehr geringen Mengen an mehrfach ungesättigten Verbindungen und Acetylenverbindungen enthalten und mindestens eines der Olefine cis-Buten-2, trans-Buten-2 und Buten-1 enthalten. Darüber hinaus können in dem Einsatzgemisch wechselnde Anteile an Isobuten zugegen sein. Solche Einsatzgemische stehen als Raffinat I, Raffinat II oder Raffinat III technisch zur Verfügung.

Die Butenhydroformylierung kann dabei in homogener Variante in dem organischen Reaktionsmedium mit gelösten Übergangsmetallkatalysatoren der Gruppe VIII des Periodensystems der Elemente nach der unmodifizierten oder der mit Komplexliganden modifizierten Variante durchgeführt werden. In dem organischen Reaktionsmedium haben sich als besonders wirksame Lösungsmittel die höher siedenden Kondensationsverbindungen der Pentanale, insbesondere die Trimeren, erwiesen, die als Nebenprodukte bei der Hydroformylierung anfallen, sowie ihre Mischungen mit den herzustellenden Pentanalen, so dass ein weiterer Lösungsmittelzusatz nicht unbedingt erforderlich ist. In einigen Fällen kann sich jedoch ein Lösungsmittelzusatz als zweckmäßig erweisen. Als Lösungsmittel werden organische Verbindungen eingesetzt, in denen Ausgangsmaterial, Reaktionsprodukt und Katalysator löslich sind. Beispiele für solche Verbindungen sind aromatische Kohlenwasserstoffe, wie Benzol und Toluol oder die isomeren Xylole und Mesitylen. Andere gebräuchliche Lösungsmittel sind Paraffinöl, Cyclohexan, n-Hexan, n-Heptan oder n-Octan, Ether wie Tetrahydrofuran, Ketone oder Texanol® der Firma Eastman. Wird bei der homogenen Variante in Gegenwart von Komplexliganden gearbeitet, eignen sich Triarylphosphine, wie Triphenylphosphin (EP 0 366 089 A2), Diphosphine, beispielsweise solche auf Basis des Xanthengerüstes (DE 102 25 282 A1), Phosphite, wie beispielsweise in US 4,599,206 beschrieben oder Diphosphite, beispielsweise wie in EP 0 213 639 A2 und DE 10 2008 002 187 A1 beschrieben. Auch Mischungen von Komplexliganden, beispielsweise aus Triarylphosphinen mit Phosphiten oder Diphosphiten, wie aus WO 2010/117391 A1 bekannt, können in der Hydroformylierungsreaktion eingesetzt werden.

Durch die Wahl der Zusammensetzung des Buteneinsatzgemisches und der Hydroformylierungsbedingungen kann das Verhältnis von n-Pentanal zu 2-Methylbutanal gesteuert werden.

Wird ein möglichst hoher Anteil an n-Pentanal gegenüber 2-Methylbutanal im Hydroformylierungsgemisch angestrebt, empfiehlt sich neben einem Buten-1 reichen Einsatzstrom die Verwendung von modifizierten Übergangsmetallkatalysatoren, die zunächst eine Isomerisierung des Restgehalts an Buten-2 in Buten-1 bewirken, das dann im überwiegenden Maße zu n-Pentanal hydroformyliert wird. In einer Ausgestaltung des erfindungsgemäßen Verfahrens können die aus DE 102 25 282 A1 bekannten Hydroformylierungskatalysatoren mit Komplexliganden auf Basis des Xanthengerüstes oder die aus EP 0 213 639 A2 oder DE 10 2008 002 187 A1 bekannten Hydroformylierungskatalysatoren auf Basis sterisch gehinderter Diphosphite eingesetzt werden.

Hohe Anteile an n-Pentanal können auch in einer zweistufigen Verfahrensvariante erhalten werden, die aus DE 101 08 474 A1 und DE 101 08 475 an sich bekannt ist. In der ersten Stufe, die ebenfalls nach der heterogenen Variante in Gegenwart von Wasser mit wasserlöslichen Komplexliganden, beispielsweise mit sulfonierten Phosphinen wie Triphenylphosphin mit unterschiedlichem Sulfonierungsgrad, durchgeführt werden kann, reagiert überwiegend Buten-1 in hoher Selektivität zu n-Pentanal und das mit Buten-2 angereicherte Abgas wird anschließend in einer zweiten Stufe unter isomersierenden Bedingungen zu einem Pentanalgemisch mit einem hohen n-Pentanalanteil umgesetzt. Durch Vereinigung der Stoffströme aus der ersten und zweiten Hydroformylierungsstufe kann ein Pentanalgemisch mit einem hohen Anteil n-Pentanal zu 2-Methylbutanal hergestellt werden.

Wird ein hoher Anteil an 2-Methylbutanal im Pentanalgemisch angestrebt, beispielsweise aufgrund von Marktgegebenheiten oder ergibt er sich aufgrund der Zusammensetzung des Buteneinsatzgemisches, arbeitet man bevorzugt in Abwesenheit von Komplexliganden nach der unmodifizierten Fahrweise. Dabei bildet sich der aktive Hydroformylierungskatalysator aus dem Übergangsmetall oder der Übergangsmetallverbindung und Kohlenmonoxid. Es wird in der Fachliteratur angenommen, dass die Übergangsmetallverbindung HM(CO)₄ die katalytisch aktive Übergangsmetallspezies bei der unmodifizierten Übergangsmetallkatalyse ist.

Mit zunehmendem Isobutengehalt in dem Buteneinsatzgemisch nimmt auch der Anteil an 3-Methylbutanal in dem Hydroformylierungsprodukt zu.

Bei der modifizierten Variante beträgt das molare Verhältnis von Übergangsmetall zu Komplexligand im Allgemeinen 1:1 bis 1:1000, es kann aber auch noch höher liegen. Bevorzugt setzt man das Übergangsmetall und den Komplexliganden in einem molaren Verhältnis von 1:3 bis 1:500, vorzugsweise 1:50 bis 1:300 ein. Die modifizierte Hydroformylierungsreaktion des Butengemisches wird üblicherweise bei Temperaturen von 50 bis 160°C und Drücken von 0,2 bis 15 MPa durchgeführt. Die Übergangsmetallkonzentration beträgt im Allgemeinen 10 bis 700 ppm, vorzugsweise 25 bis 500 ppm, bezogen auf die Reaktionsmischung.

Wird nach der unmodifizierten Variante gearbeitet, setzt man das Übergangsmetall in geringeren Mengen ein, im Allgemeinen in einer Menge von 1 bis 100 ppm, vorzugsweise 2 bis 30 ppm, bezogen auf die eingesetzte Butenmenge. Man arbeitet zweckmäßigerweise bei höheren Drücken im Bereich von 5 bis 70 MPa, vorzugsweise von 5 bis 60 MPa und insbesondere von 10 bis 30 MPa. Geeignete Reaktionstemperaturen bewegen sich im Bereich von 50 bis 180°C, bevorzugt von 50 bis 150°C und insbesondere von 100 bis 150°C.

Die Zusammensetzung des Synthesegases kann in weiten Grenzen variiert werden. Im Allgemeinen setzt man Gemische ein, in denen das Molverhältnis von Kohlenmonoxid zu Wasserstoff 5:1 bis 1:5 beträgt. Üblicherweise ist dieses Verhältnis 1:1 oder weicht von diesem Wert zugunsten von Wasserstoff nur wenig ab. Das Gemisch enthaltend lineare Butene kann als solches oder in Lösung mit organischen Lösungsmitteln, wie Kohlenwasserstoffen, der Reaktionszone zugeführt werden.

Vorzugsweise verwendet man als Übergangsmetalle der Gruppe VIII des Periodensystems der Elemente Kobalt, Rhodium, Iridium, Nickel, Palladium, Platin, Eisen oder Ruthenium und insbesondere Rhodium und Kobalt. Der modifizierte oder nicht modifizierte Übergangsmetallkatalysator bildet sich unter den Bedingungen der Hydroformylierungsreaktion aus den eingesetzten Übergangsmetallverbindungen, wie deren Salzen, wie Chloriden, Nitraten, Sulfaten, Acetaten, Pentanoaten oder 2-Ethylhexanoaten, deren Chalkogeniden, wie Oxiden oder Sulfiden, deren Carbonylverbindungen, wie M₂(CO)₈, M₄(CO)₁₂, M₆(CO)₁₆, M₂(CO)₉, M₃(CO)₁₂, deren Organoübergangsmetallverbindungen, wie Carbonylacetylacetonaten oder Cyclooctadienylacetaten oder -chloriden. Dabei kann die Übergangsmetallverbindung als Feststoff oder zweckmäßigerweise in Lösung eingesetzt werden. Als Übergangsmetallverbindung, die als Katalysatorvorstufe verwendet wird, eignet sich insbesondere Rhodiumpentanoat, Rhodiumacetat, Rhodium-2-ethyl-hexanoat oder Kobaltpentanoat, Kobaltacetat oder Kobalt-2-ethylhexanoat oder Co₂(CO)₈, Co₄(CO)₁₂, Rh₂(CO)₈, Rh₄(CO)₁₂ oder Rh₆(CO)₁₆ oder Cyclopentadienylrhodiumverbindungen, Rhodiumacetylacetonat oder Rhodiumdicarbonylacetylacetonat. Bevorzugt werden Rhodiumoxid und insbesondere Rhodiumacetat, Rhodium-2-ethylhexanoat und Rhodiumpentanoat eingesetzt.

Es ist aber auch möglich, den Übergangsmetallkatalysator in einer Vorcarbonylierungsstufe zunächst zu präformieren und ihn anschließend der eigentlichen Hydroformylierungsstufe zuzuführen. Die Bedingungen der Präformierung entsprechen dabei im Allgemeinen den Hydroformylierungsbedingungen.

Die Hydroformylierungsstufe kann sowohl diskontinuierlich als auch kontinuierlich durchgeführt werden. Die Abtrennung des gebildeten Pentanalgemisches von dem Hydroformylierungskatalysator erfolgt nach konventionellen Verfahren, beispielsweise durch Destillation bei der homogenen Verfahrensführung oder durch einfache Phasentrennung von der wässrigen Katalysatorlösung bei der heterogenen oder zweiphasigen Verfahrensführung.
Der Übergangsmetallkatalysator wird gegebenenfalls nach Zusatz von frischer Übergangsmetallverbindung sowie gegebenenfalls von Frischligand, falls nach der modifizierten Fahrweise gearbeitet wird, und nach Entnahme eines Teils der im Verlauf der Reaktion gebildeten Aldehydkondensationsprodukte in die Reaktionszone zurückgeführt.

Die Forderung nach einer bestimmten Zusammensetzung des erhaltenen Pentanalgemisches bezüglich der Isomeren n-Pentanal, 2-Methylbutanal und 3-Methylbutanal richtet sich nach den Marktgegebenheiten und kann durch die Zusammensetzung des Einsatzbutengemisches sowie durch die Wahl der Hydroformylierungsbedingungen gesteuert werden. Häufig wird ein Pentanalgemisch angestrebt, das im Allgemeinen mindestens 85 mol-% n-Pentanal, weniger als 15 mol-% 2-Methylbutanal und in Abhängigkeit vom Isobutengehalt weniger als 5 mol-% 3-Methylbutanal, vorzugsweise weniger als 1 mol-% und insbesondere weniger als 0,2 mol-% 3-Methylbutanal enthält, jeweils bezogen auf die Summe an Pentanalen. Aber auch Pentanalgemische mit einem höheren Anteil an 2-Methylbutanal können vom Markt gefordert werden.

Das nach der Hydroformylierungsstufe und nach Katalysatorabtrennung erhaltene Pentanalgemisch, das man auch als rohes Hydroformylierungsprodukt ansehen kann, wird anschließend in einen mit 2-Methylbutanal und an 3-Methylbutanal angereicherten leichter flüchtigen Stoffstrom und in einen mit n-Pentanal angereicherten schwerer flüchtigen Stoffstrom aufgetrennt, zweckmäßigerweise durch Destillation. Die Destillation des rohen Hydroformylierungsprodukts erfolgt nach konventionellen Verfahren an einer Destillationskolonne. Um möglichst reines n-Pentanal im schwerer flüchtigen Stoffstrom anzureichern, wird die Trennschärfe in der Destillation im Allgemeinen so gewählt, dass der erhaltene mit 2-Methylbutanal und mit 3-Methylbutanal angereicherte leichter flüchtige Stoffstrom ebenfalls noch Mengen an n-Pentanal enthält. Seine genaue Zusammensetzung hängt von der Zusammensetzung des Einsatzbutengemisches, den Hydroformylierungsbedingungen und den Destillationsbedingungen ab, beispielsweise kann die Zusammensetzung dieses flüchtigen Stoffstromes bei 80 bis 85 mol-% 2-Methylbutanal, 10 bis 14 mol-% n-Pentanal und 1 bis 10 mol-% 3-Methylbutanal, bezogen auf den Pentanalgehalt, liegen. Je nach Isobutengehalt im Einsatzbutengemisch kann der 3-Methylbutanalgehalt aber auch höher oder niedriger sein. Im schwerer flüchtigen Stoffstrom wird n-Pentanal nahezu frei von den übrigen Pentanalisomeren aufkonzentriert.

Während n-Pentanal mit einem Siedepunkt von 103°C bei Normaldruck aus diesem flüchtigen Stoffstrom durch eine weitere konventionelle Destillation an einer weiteren Kolonne mit 10 bis 100 Böden als Produkt hoher Reinheit abgetrennt werden kann, besitzen 2-Methylbutanal, das einen Siedepunkt von 92°C bei Normaldruck hat, und 3-Methylbutanal, das bei Normaldruck bei 92,5°C siedet, eine zu geringe Siedepunktdifferenz für eine ausreichende Trennung bei vertretbarem Destillationsaufwand. Diese weitere Destillation zur n-Pentanalabtrennung kann optional durchgeführt werden.

Erfindungsgemäß wird der nach Destillation des rohen Hydroformylierungsprodukts erhaltene leichter flüchtige Stoffstrom mit dem Hauptbestandteil 2-Methylbutanal und den Restgehalten an n-Pentanal und 3-Methylbutanal mit Formaldehyd versetzt. Die Behandlung von α-alkylsubstituierten Aldehyden mit Formaldehyd zur Entfernung von Restmengen an Aldehyden mit zwei Wasserstoffatomen am α-ständigen Kohlenstoffatom relativ zur Carbonylgruppe im Zuge der Aufreinigung wird auch als Methylenierungsreaktion bezeichnet und ist aus dem Stand der Technik an sich bekannt und wird beispielsweise in DE 3842186 A1 und DE 3744212 A1 beschrieben.

Das 2-Methylbutanal, 3-Methylbutanal und n-Pentanal enthaltende Gemisch wird mit Formaldehyd umgesetzt in Gegenwart eines Aldolisierungskatalysators, typischerweise mit einem Gemisch aus einem sekundären Amin, beispielsweise einem Amin der allgemeinen Formel R¹-NH-R², wobei R¹ und R² gleich oder verschieden sind und für Akylreste mit 1 bis 12, vorzugsweise 3 bis 5 Kohlenstoffatomen stehen, und einer Monocarbonsäure mit 1 bis 10 Kohlenstoffatomen oder einer Di- oder Polycarbonsäure mit 2 bis 10 Kohlenstoffatomen. Vorzugsweise verwendet man als Aldolisierungskatalysator ein Gemisch aus Di-n-butylamin und n-Buttersäure. Aber auch andere Aldolisierungskatalysatoren sind möglich. Formaldehyd wird in fester Form, als Paraformaldehyd, oder zweckmäßigerweise als wässrige Lösung in handelsüblicher Konzentration, wie 30 bis 50 Gew.-%ig, verwendet, wobei das molare Verhältnis von Formaldehyd zu der Summe an Aldehyden mit zwei Wasserstoffatomen am α-ständigen Kohlenstoffatom relativ zur Carbonylgruppe 1 bis 2 beträgt.

Die Umsetzung wird üblicherweise bei Temperaturen von 0 bis 100°C bei Eigendruck oder leichtem Überdruck durchgeführt. Als Reaktoren eignen sich die in der chemischen Verfahrenstechnik üblichen Aggregate, wie Rührkessel, Rührkesselkaskaden, Mischpumpen oder Strömungsrohre. Sowohl eine absatzweise als auch kontinuierliche Reaktionsführung ist möglich. Besonders eignen sich für die kontinuierliche Reaktionsführung Strömungsrohre, beispielsweise ein senkrechts stehendes oder ein waagerecht angeordnetes Strömungsrohr oder ein mehrfach geschlägeltes Strömungsrohr. Das Strömungsrohr kann als Leerrohr betrieben werden, es kann jedoch ebenfalls Füllkörper oder Einbauten zur intensiven Durchmischung der organischen Phase mit der wässrigen Phase enthalten, beispielsweise Raschigringe, Sättel, Pallringe, Wendel, Strombrecher oder statische Mischer oder Mischerpackungen. Statische Mischelemente stehen kommerziell zur Verfügung und werden beispielsweise als Sulzermischer oder Kenicksmischer angeboten. Es hat sich eine Belastung des Strömungsrohrs mit dem Gemisch aus organischer Phase enthaltend 2-Methylbutanal, 3-Methylbutanal sowie n-Pentanal und wässriger Formaldehydlösung von 0,1 bis 10 h⁻¹, vorzugsweise von 0,1 bis 5 h⁻¹, und insbesondere von 0,1 bis 3h⁻¹ bezogen auf Reaktorvolumen und Zeit, als zweckmäßig erwiesen.

Das das Reaktionsgefäß verlassende Gemisch wird in einen Phasentrenner geleitet, in dem sich die organische Phase von der wässrigen Phase trennt.

In einer besonderen Verfahrensvariante wird das Gemisch enthaltend 2-Methylbutanal, 3-Methylbutanal und n-Pentanal in Gegenwart von Formaldehyd und den Aldolisierungskatalysator destilliert, wobei nahezu 3-methylbutanalfreies und n-pentanalfreies 2-Methylbutanal als Hauptfraktion abdestilliert. Neben der organischen Phase fällt Wasser durch den Wassereintrag bei Verwendung einer wässrigen Formaldehydlösung und durch die Bildung von n-Propylacrolein und Isopropylacrolein an. Bei der Methylenierung bildet sich zunächst aus Formaldehyd und dem sekundären Amin im sauren Medium ein Mannich-Salz, das bevorzugt mit 3-Methylbutanal und n-Pentanal über die Bildung des α-Methylolderivats in Isopropylacrolein und n-Propylacrolein dehydratisiert, während 2-Methylbutanal unverändert bleibt.

Die abgetrennte organische Phase enthaltend nicht reagiertes 2-Methylbutanal, n-Propylacrolein und Isopropylacrolein wird anschließend in Gegenwart von Hydrierkatalysatoren mit Wasserstoff bei erhöhter Temperatur und erhöhtem Druck selektiv hydriert. Dabei wird die Doppelbindung in der α,β-Position relativ zum Carbonylkohlenstoffatom unter Beibehaltung der Aldehydgruppe abgesättigt, so dass als Ergebnis der Selektivhydrierung ein Gemisch enthaltend unverändertes 2-Methylbutanal und isomere Hexanale, wie 2-Methylpentanal und 2,3-Dimethylbutanal, erhalten wird. Die Selektivhydrierung wird in bekannter Weise an geträgerten oder ungeträgerten Katalysatoren, die als hydrieraktive Komponente Palladium, Platin, Rhodium und/oder Nickel enthalten, durchgeführt. Vorzugsweise arbeitet man mit Palladiumkatalysatoren bei Temperaturen von 120 bis 180°C, vorzugsweise 140 bis 160°C und bei einem Druck von 1,5 bis 5 MPa, vorzugsweise bei 2 bis 3 MPa.

Der erhaltene Hydrieraustrag wird anschließend in einen mit 2-Methylbutanal angereicherten Stoffstrom und in einen mit dem Gemisch isomerer Hexanale angereicherten Stoffstrom aufgetrennt, zweckmäßiger Weise durch Destillation.

Anschließend wird das Gemisch isomerer Hexanale zu einem Gemisch isomerer Hexansäure enthaltend 2-Methylpentansäure und 2,3-Dimethylbuttersäure oxidiert. Die Oxidation von α-methylverzweigten Aldehyden ist an sich bekannt (Ullmann's Encyclopedia of Industrial Chemistry, 6th. Edition, 2003, Wiley-VCH Verlag GmbH & Co. KGaA, Weinheim, Vol. 6, Seiten 497-502) und wird vorzugsweise in der Flüssigphase durchgeführt, beispielsweise in Rohrrektoren, die mit einem Anströmboden versehen sind, obwohl andere Verfahrensausgestaltungen wie die Oxidation in der Gasphase nicht ausgeschlossen sind. Als Oxidationsmittel eignen sich übliche, zur Oxidation von aliphatischen Aldehyden geeignete Verbindungen wie Sauerstoff, sauerstoffenthaltende Gasgemische, Ozon, ozonhaltige Gasgemische, Peroxide, Persäuren, Metallsalze von Persäuren oder Übergangsmetalle in hohen Oxidationsstufen, beispielsweise Kaliumpermanganat oder Braunstein. Aufgrund der guten Verfügbarkeit verwendet man als Oxidationsmittel zweckmäßig molekularen Sauerstoff oder Gasgemische, die molekularen Sauerstoff enthalten. Weitere Bestandteile derartiger Gasgemische sind inerte Gase, z. B. Stickstoff, Edelgase und Kohlendioxid. Der Anteil der inerten Bestandteile des sauerstoffenthaltenden Gasgemisches beträgt bis zu 90 Vol-%, insbesondere 30 bis 80 Vol-%. Die bevorzugten Oxidationsmittel sind Sauerstoff oder Luft.

Die Oxidation kann entweder unter Katalysatorzusatz oder in Abwesenheit von Katalysatoren durchgeführt werden. Als Katalysatoren eignen sich Übergangsmetalle oder Verbindungen von Übergangsmetallen, die in geringen Mengen wie beispielsweise von 0,1 bis 5 ppm, berechnet als Übergangsmetall und bezogen auf eingesetzten Aldehyd, zugesetzt werden können wie Titan, Vanadium, Chrom, Molybdän, Mangan, Eisen, Kobalt, Nickel, Ruthenium, Rhodium, Palladium oder Kupfer. Eine solche Verfahrensführung wird beispielsweise in DE 100 10 771 C1 oder DE 26 04 545 A1 beschrieben.

Ebenfalls kann die Umsetzung in Anwesenheit von Alkali- oder Erdalkalimetallsalzen schwacher Säuren durchgeführt werden. Insbesondere bei der Oxidation von α-verzweigten Aldehyden, bei denen das dem Carbonylkohlenstoffatom benachbarte Kohlenstoffatom die Verzweigung trägt, empfiehlt der Stand der Technik die Anwesenheit von geringen Mengen an Alkalimetallcarboxylaten zur Selektivitätsverbesserung (DE 950 007, DE 100 10 771 C1). Zweckmäßigerweise wird die Oxidation in Gegenwart von 1 bis 30 mmol, vorzugsweise 1 bis 15 mmol und insbesondere 1 bis 8 mmol, je Mol Aldehyd, berechnet als Alkali- oder Erdalkalimetall, durchgeführt. Auch kann eine Kombination von Alkali- oder Erdalkalimetallcarboxylaten mit Übergangsmetallverbindungen, wie in EP 1 854 778 A1 behandelt, verwendet werden.

Es ist nicht erforderlich, die Alkali- oder Erdalkalimetallcarboxylate als einheitliche Verbindung einzusetzen. Es ist ebenfalls möglich, Gemische dieser Verbindungen zu verwenden, wobei man jedoch zweckmäßigerweise 2-Methylpentanoate verwendet. Vorzugsweise setzt man jedoch einheitliche Verbindungen ein, beispielsweise Lithium-, Kalium-, Natrium-, Calcium- oder Barium-2-Methylpentanoat.

Im Allgemeinen stellt man eine Alkali- oder Erdalkalimetall-2-Methylpentanoate enthaltende Lösung durch Neutralisation einer wässrigen, die Alkali- oder Erdalkalimetallverbindung enthaltenden Lösung mit einem Überschuss an 2-Methylpentansäure her und setzt diese Lösung dem zu oxidierenden Gemisch aus den isomeren Hexanalen zu. Als Alkali- oder Erdalkalimetallverbindungen eignen sich besonders die Hydroxide, Carbonate oder Hydrogencarbonate.

Es ist aber auch möglich, die Alkali- oder Erdalkalimetall-2-Methylpentanoate im Reaktionsgemisch zu erzeugen, indem man Alkali- oder Erdalkalimetallverbindungen zusetzt, die unter den Reaktionsbedingungen in die 2-Methylpentanoate überführt werden. Beispielsweise lassen sich Alkali- oder Erdalkalimetallhydroxide, -carbonate, -hydrogencarbonate oder -oxide in der Oxidationsreaktion einsetzen. Ihr Zusatz kann entweder in fester Form oder als wässrige Lösung erfolgen.

Die Umsetzung mit dem Oxidationsmittel, vorzugsweise mit Sauerstoff oder sauerstoffenthaltenden Gasen, wird in einem Temperaturbereich von 20 bis 100°C durchgeführt. Vorzugsweise arbeitet man zwischen 20 und 80°C, insbesondere zwischen 40 und 80°C. Die Temperaturführung, konstante oder variable Temperatur, kann den individuellen Erfordernissen des Ausgangsmaterials und den Reaktionsgegebenheiten angepasst werden.

Die Umsetzung der Reaktionspartner erfolgt bevorzugt bei Atmosphärendruck. Die Anwendung erhöhten Drucks ist jedoch nicht ausgeschlossen. Üblicherweise arbeitet man in einem Bereich von Atmosphärendruck bis 1,5 MPa, vorzugsweise bei Atmosphärendruck bis 0,8 MPa.

Der Oxidationsschritt kann absatzweise oder kontinuierlich durchgeführt werden. Eine Rückführung nicht umgesetzter Reaktionsteilnehmer ist in beiden Fällen möglich.

Das erhaltene Rohsäuregemisch enthaltend die isomeren Hexansäuren wird anschließend zu spezifikationsgerechter Ware in üblicher Weise fraktioniert aufdestilliert.

Im Allgemeinen wird ein Gemisch isomerer Hexansäuren in einer Reinheit von im Allgemeinen mehr als 95 Gew.-% bei einer Siedetemperatur im Bereich von 191 bis 196°C unter Normaldruck erhalten. Das Gemisch isomerer Hexansäuren enthält überwiegend 2-Methylpentansäure und in Abhängigkeit von dem Isobutengehalt im Buteneinsatzgemisch 2,3-Dimethylbuttersäure und eignet sich in hervorragender Weise als Säurekomponente für die Herstellung von Esterschmierstoffen und Riechstoffen.

In einer weiteren Ausgestaltung des erfindungsgemäßen Verfahrens wird nach der Hydroformylierung des Einsatzbutengemisches enthaltend lineare Butene, der Abtrennung des gebildeten Pentanalgemisches von dem Hydroformylierungskatalysator, der Abtrennung eines mit 2-Methylbutanal und 3-Methylbutanal angereicherten leichter flüchtigen Stoffstromes, der noch Restmengen an n-Pentanal enthält, von einem mit n-Pentanal angereicherten schwerer flüchtigen Stoffstromes, der Behandlung des leichter flüchtigen Stoffstromes mit Formaldehyd in Gegenwart eines Aldolisierungskatalysators, wie zuvor beschrieben, die organische Phase von der wässrigen Phase abgetrennt und die rohe organische Phase unter konventionellen Bedingungen aufdestilliert, wobei nicht umgesetztes 2-Methylbutanal als leichter flüchtige Komponente vom n-Propylacrolein und vom Isopropylacrolein abgetrennt wird.

Die schwerer siedende Fraktion enthaltend n-Propylacrolein und Isopropylacrolein wird, wie zuvor beschrieben, in Gegenwart eines Hydrierkatalysators bei erhöhter Temperatur und erhöhtem Druck mit Wasserstoff selektiv in ein Gemisch isomerer Hexanale, enthaltend 2-Methylpentanal und 2,3-Dimethylbutanal, umgewandelt. Das so erhaltene Gemisch isomerer Hexanale wird anschließend, wie zuvor beschrieben, oxidiert. Aus der angefallenenen Rohsäure wird destillativ unter konventionellen Bedingungen ein gereinigtes Gemisch isomerer Hexansäuren mit einem überwiegenden Anteil an 2-Methylpentansäure erhalten.

Die vorliegende Erfindung betrifft daher ebenfalls ein Verfahren zur Herstellung von isomeren Hexansäuren enthaltend 2-Methylpentansäure aus den bei der Herstellung von Pentanalen anfallenden Nebenströmen, dadurch gekennzeichnet, dass man
a) ein Gemisch enthaltend lineare Butene in Gegenwart von Übergangsmetallverbindungen der Gruppe VIII des Periodensystems der Elemente mit Kohlenmonoxid und Wasserstoff bei erhöhter Temperatur und erhöhtem Druck zu einem Pentanalgemisch umsetzt;
b) das gemäß Schritt a) erhaltene Gemisch in einen mit 2-Methylbutanal und 3-Methylbutanal angereicherten Stoffstrom und in einen mit n-Pentanal angereicherten Stoffstrom trennt;
c) den gemäß Schritt b) mit 2-Methylbutanal und 3-Methylbutanal angereicherten Stoffstrom mit Formaldehyd umsetzt;
d) aus dem nach Schritt c) erhaltenen Reaktionsgemisch einen mit 2-Methylbutanal angereicherten und einen mit 2-Methylbutanal abgereicherten Stoffstrom gewinnt;
e) den nach Schritt d) erhaltenen mit 2-Methylbutanal abgereicherten Stoffstrom in Gegenwart eines Hydrierkatalysators mit Wasserstoff bei erhöhter Temperatur und erhöhtem Druck selektiv zu einem Gemisch enthaltend isomere Hexanale umsetzt; und
f) das gemäß Schritt e) erhaltene Reaktionsgemisch enthaltend isomere Hexanale oxidiert und ein Gemisch isomerer Hexansäuren enthaltend 2-Methylpentansäure destillativ abtrennt.

Die nach den erfindungsgemäßen Verfahren erhaltenen 2-methylbutanalhaltigen Stoffströme sind überwiegend frei von den übrigen Pentanalisomeren und können nach an sich bekannten Verfahren in 2-Methylbutanol oder 2-Methylbutylamine derivatisiert werden. (Ullmann's Encyclopedia of Industrial Chemistry, 6. Auflage, 2003 Wiley-VCH Verlag, Band 2, Seiten 21-24, Seiten 384-388). Sie können ebenfalls durch eine Oxidation, wie zuvor beschrieben, in 2-Methylbuttersäure umgewandelt werden. Die Reinheit der erhaltenen 2-Methylbuttersäure liegt im Allgemeinen bei über 99,7 Gew.%, bezogen auf den organischen Anteil. 2-Methylbuttersäure dieser Qualität eignet sich in hervorragender Weise als Säurekomponente für die Herstellung von Esterschmierstoffen und Riechstoffen. Sie kann ebenfalls mit hochreiner n-Pentansäure zu einem hochreinen Säuregemisch, das man auch als iso-Pentansäure bezeichnen kann, verarbeitet werden.

Ist 2-Methylbuttersäure das Zielprodukt der Derivatisierung von 2-Methyl-butanal, so kann in einer sehr speziellen Ausführungsform der Erfindung nach der Hydroformylierung des Einsatzbutengemisches enthaltend lineare Butene, der Abtrennung des gebildeten Pentanalgemisches von dem Hydroformylierungskatalysator, der Abtrennung eines mit 2-Methylbutanal und 3-Methylbutanal angereicherten leichter flüchtigen Stoffstromes, der noch Restmengen an n-Pentanal enthält, von einem mit n-Pentanal angereicherten schwerer flüchtigen Stoffstromes, der Behandlung des leichter flüchtigen Stoffstromes mit Formaldehyd in Gegenwart eines Aldolisierungskatalysators und der Selektivhydrierung des Methylenierungsproduktes, wie zuvor beschrieben, zu einem Gemisch enthaltend 2-Methylbutanal und isomere Hexanale, das erhaltene Reaktionsprodukt gemäß den zuvor beschriebenen Bedingungen oxidiert werden. Aus dem erhaltenen Säuregemisch kann 2-Methylbuttersäure mit einem Siedepunkt von 177°C/Normaldruck und das Gemisch isomerer Hexansäuren enthaltend 2-Methylpentansäure mit einem Siedebereich von 191 bis 196°C/Normaldruck in hoher Reinheit fraktioniert destilliert werden.

Die vorliegende Erfindung betrifft daher ebenfalls ein Verfahren zur Herstellung von isomeren Hexansäuren enthaltend 2-Methylpentansäure aus den bei der Herstellung von Pentanalen anfallenden Nebenströmen, dadurch gekennzeichnet, dass man
a) ein Gemisch enthaltend lineare Butene in Gegenwart von Übergangsmetallverbindungen der Gruppe VIII des Periodensystems der Elemente mit Kohlenmonoxid und Wasserstoff bei erhöhter Temperatur und erhöhtem Druck zu einem Pentanalgemisch umsetzt;
b) das gemäß Schritt a) erhaltene Gemisch in einen mit 2-Methylbutanal und 3-Methylbutanal angereicherten Stoffstrom und in einen mit n-Pentanal angereicherten Stoffstrom trennt;
c) den gemäß Schritt b) mit 2-Methylbutanal und 3-Methylbutanal angereicherten Stoffstrom mit Formaldehyd umsetzt;
d) das nach Schritt c) erhaltene Reaktionsgemisch in Gegenwart eines Hydrierkatalysators mit Wasserstoff bei erhöhter Temperatur und erhöhtem Druck selektiv zu einem Gemisch enthaltend 2-Methylbutanal und isomere Hexanale umsetzt; und
e) das gemäß Schritt d) erhaltene Reaktionsgemisch oxidiert und anschließend 2-Methylbuttersäure und ein Gemisch isomerer Hexansäuren enthaltend 2-Methylpentansäure destillativ abtrennt.

Das erfindungsgemäße Verfahren ermöglicht es, die bei der Pentanalherstellung anfallenden Nebenströme in isomere Hexansäuren wie 2-Methylpentansäure und 2,3-Dimethylbuttersäure zu derivatisieren und die Wirtschaftlichkeit des Verfahrens zur Pentanalherstellung zu verbessern.

Ebenfalls können die bei der Durchführung der verschiedenen Ausführungsformen der Erfindung erhaltenen Gemische isomerer Hexanale enthaltend überwiegend 2-Methylpentanal auch in ein Gemisch isomerer Hexanole enthaltend überwiegend 2-Methylpentanol oder in ein Gemisch isomerer Hexylamine derivatisiert werden. Derartige Derivatisierungsreaktionen von Aldehyden sind an sich bekannt und werden beispielsweise im Ullmann's Encyclopedia of Industrial Chemistry, 6. Auflage, 2003 Wiley-VCH Verlag, Band 2, Seiten 21-24; Seiten 384-388, beschrieben.

## Patentansprüche

1. Verfahren zur Herstellung von isomeren Hexansäuren enthaltend 2-Methylpentansäure aus den bei der Herstellung von Pentanalen anfallenden Nebenströmen, **dadurch gekennzeichnet, dass** man
a) ein Gemisch enthaltend lineare Butene in Gegenwart von Übergangsmetallverbindungen der Gruppe VIII des Periodensystems der Elemente mit Kohlenmonoxid und Wasserstoff bei erhöhter Temperatur und erhöhtem Druck zu einem Pentanalgemisch umsetzt;
b) das gemäß Schritt a) erhaltene Gemisch in einen mit 2-Methylbutanal und 3-Methylbutanal angereicherten Stoffstrom und in einen mit n-Pentanal angereicherten Stoffstrom trennt;
c) den gemäß Schritt b) mit 2-Methylbutanal und 3-Methylbutanal angereicherten Stoffstrom mit Formaldehyd umsetzt;
d) das nach Schritt c) erhaltene Reaktionsgemisch in Gegenwart eines Hydrierkatalysators mit Wasserstoff bei erhöhter Temperatur und erhöhtem Druck selektiv zu einem Gemisch enthaltend 2-Methylbutanal und isomere Hexanale umsetzt;
e) das gemäß Schritt d) erhaltene Reaktionsgemisch in einen mit 2-Methylbutanal angereicherten Stoffstrom und in einen mit einem Gemisch isomerer Hexanale angereicherten Stoffstrom trennt; und
f) das gemäß Schritt e) erhaltene Gemisch isomerer Hexanale zu einem Gemisch isomerer Hexansäuren enthaltend 2-Methylpentansäure oxidiert.

2. Verfahren zur Herstellung von isomeren Hexansäuren enthaltend 2-Methylpentansäure aus den bei der Herstellung von Pentanalen anfallenden Nebenströmen, **dadurch gekennzeichnet, dass** man
a) ein Gemisch enthaltend lineare Butene in Gegenwart von Übergangsmetallverbindungen der Gruppe VIII des Periodensystems der Elemente mit Kohlenmonoxid und Wasserstoff bei erhöhter Temperatur und erhöhtem Druck zu einem Pentanalgemisch umsetzt;
b) das gemäß Schritt a) erhaltene Gemisch in einen mit 2-Methylbutanal und 3-Methylbutanal angereicherten Stoffstrom und in einen mit n-Pentanal angereicherten Stoffstrom trennt;
c) den gemäß Schritt b) mit 2-Methylbutanal und 3-Methylbutanal angereicherten Stoffstrom mit Formaldehyd umsetzt;
d) aus dem nach Schritt c) erhaltenen Reaktionsgemisch einen mit 2-Methylbutanal angereicherten und einen mit 2-Methylbutanal abgereicherten Stoffstrom gewinnt;
e) den nach Schritt d) erhaltenen mit 2-Methylbutanal abgereicherten Stoffstrom in Gegenwart eines Hydrierkatalysators mit Wasserstoff bei erhöhter Temperatur und erhöhtem Druck selektiv zu einem Gemisch enthaltend isomere Hexanale umsetzt; und
f) das gemäß Schritt e) erhaltene Reaktionsgemisch oxidiert und isomere Hexansäuren destillativ abtrennt.

3. Verfahren zur Herstellung von isomeren Hexansäuren enthaltend 2-Methylpentansäure aus den bei der Herstellung von Pentanalen anfallenden Nebenströmen, **dadurch gekennzeichnet, dass** man
a) ein Gemisch enthaltend lineare Butene in Gegenwart von Übergangsmetallverbindungen der Gruppe VIII des Periodensystems der Elemente mit Kohlenmonoxid und Wasserstoff bei erhöhter Temperatur und erhöhtem Druck zu einem Pentanalgemisch umsetzt;
b) das gemäß Schritt a) erhaltene Gemisch in einen mit 2-Methylbutanal und 3-Methylbutanal angereicherten Stoffstrom und in einen mit n-Pentanal angereicherten Stoffstrom trennt;
c) den gemäß Schritt b) mit 2-Methylbutanal und 3-Methylbutanal angereicherten Stoffstrom mit Formaldehyd umsetzt;
d) das nach Schritt c) erhaltene Reaktionsgemisch in Gegenwart eines Hydrierkatalysators mit Wasserstoff bei erhöhter Temperatur und erhöhtem Druck selektiv zu einem Gemisch enthaltend 2-Methylbutanal und isomere Hexanale umsetzt; und
e) das gemäß Schritt d) erhaltene Reaktionsgemisch oxidiert und anschließend 2-Methylbuttersäure und ein Gemisch isomerer Hexansäuren enthaltend 2-Methylpentansäure destillativ abtrennt.

4. Verfahren gemäß einem der Ansprüche 1 bis 3, **dadurch gekennzeichnet, dass** die Umsetzung mit Formaldehyd gemäß Schritt c) in Gegenwart eines sekundären Amins und einer Mono-, Di- oder Polycarbonsäure erfolgt.

5. Verfahren gemäß einem oder mehreren der Ansprüche 1 bis 4, **dadurch gekennzeichnet, dass** man während der Umsetzung mit Formaldehyd gemäß Schritt c) destilliert.

6. Verfahren gemäß einem oder mehreren der Ansprüche 1 bis 5, **dadurch gekennzeichnet, dass** man als sekundäres Amin ein Alkylamin der Formel R¹-NH-R² verwendet, wobei R¹ und R² gleich oder verschieden sind und für Akylreste mit 1 bis 12, vorzugsweise 3 bis 5 Kohlenstoffatomen stehen.

7. Verfahren gemäß einem oder mehreren der Ansprüche 1 bis 6, **dadurch gekennzeichnet, dass** man eine Monocarbonsäure mit 1 bis 10 Kohlenstoffatomen oder eine Di- oder Polycarbonsäure mit 2 bis 10 Kohlenstoffatomen verwendet.

8. Verfahren gemäß einem oder mehreren der Ansprüche 1 bis 7, **dadurch gekennzeichnet, dass** die Oxidation in Anwesenheit von Alkali- oder Erdalkalimetallcarboxylaten erfolgt.

9. Verfahren gemäß Anspruch 8, **dadurch gekennzeichnet, dass** man als Alkali- oder Erdalkalimetallcarboxylate Lithium-, Kalium-, Natrium-, Calcium- oder Bariumcarboxylat verwendet.

10. Verfahren gemäß einem oder mehreren der Ansprüche 1 bis 9, **dadurch gekennzeichnet, dass** man die Oxidation in der Flüssigphase durchführt.

11. Verfahren gemäß einem oder mehreren der Ansprüche 1 bis 10, **dadurch gekennzeichnet, dass** man als Oxidationsmittel Sauerstoff oder sauerstoffenthaltende Gase verwendet.

12. Verfahren gemäß einem oder mehreren der Ansprüche 1 bis 11, **dadurch gekennzeichnet, dass** man die Umsetzung mit Formaldehyd kontinuierlich in einem Strömungsrohr durchführt.

## Claims

1. Process for preparing isomeric hexanoic acids comprising 2-methylpentanoic acid from the secondary streams obtained in the preparation of pentanals, **characterized in that**
a) a mixture comprising linear butenes is reacted in the presence of transition metal compounds of group VIII of the Periodic Table of the Elements with carbon monoxide and hydrogen at elevated temperature and elevated pressure to give a pentanal mixture;
b) the mixture obtained in step a) is separated into a stream enriched with 2-methylbutanal and 3-methyl-butanal, and a stream enriched with n-pentanal;
c) the stream enriched with 2-methylbutanal and 3-methylbutanal of step b) is reacted with formaldehyde;
d) the reaction mixture obtained after step c) is selectively reacted in the presence of a hydrogenation catalyst with hydrogen at elevated temperature and elevated pressure to give a mixture comprising 2-methylbutanal and isomeric hexanals;
e) the reaction mixture obtained in step d) is separated into a stream enriched with 2-methylbutanal and a stream enriched with a mixture of isomeric hexanals; and
f) the mixture of isomeric hexanals obtained in step e) is oxidized to a mixture of isomeric hexanoic acids comprising 2-methylpentanoic acid.

2. Process for preparing isomeric hexanoic acids comprising 2-methylpentanoic acid from the secondary streams obtained in the preparation of pentanals, **characterized in that**
a) a mixture comprising linear butenes is reacted in the presence of transition metal compounds of group VIII of the Periodic Table of the Elements with carbon monoxide and hydrogen at elevated temperature and elevated pressure to give a pentanal mixture;
b) the mixture obtained in step a) is separated into a stream enriched with 2-methylbutanal and 3-methyl-butanal, and a stream enriched with n-pentanal;
c) the stream enriched with 2-methylbutanal and 3-methylbutanal of step b) is reacted with formaldehyde;
d) a stream enriched with 2-methylbutanal and a stream depleted of 2-methylbutanal are recovered from the reaction mixture obtained after step c);
e) the stream depleted of 2-methylbutanal obtained after step d) is selectively reacted in the presence of a hydrogenation catalyst with hydrogen at elevated temperature and elevated pressure to give a mixture comprising isomeric hexanals; and
f) the reaction mixture obtained in step e) is oxidized and isomeric hexanoic acids are removed by distillation.

3. Process for preparing isomeric hexanoic acids comprising 2-methylpentanoic acid from the secondary streams obtained in the preparation of pentanals, **characterized in that**
a) a mixture comprising linear butenes is reacted in the presence of transition metal compounds of group VIII of the Periodic Table of the Elements with carbon monoxide and hydrogen at elevated temperature and elevated pressure to give a pentanal mixture;
b) the mixture obtained in step a) is separated into a stream enriched with 2-methylbutanal and 3-methyl-butanal, and a stream enriched with n-pentanal;
c) the stream enriched with 2-methylbutanal and 3-methylbutanal of step b) is reacted with formaldehyde;
d) the reaction mixture obtained after step c) is selectively reacted in the presence of a hydrogenation catalyst with hydrogen at elevated temperature and elevated pressure to give a mixture comprising 2-methylbutanal and isomeric hexanals; and
e) the reaction mixture obtained in step d) is oxidized and then 2-methylbutyric acid and a mixture of isomeric hexanoic acids comprising 2-methylpentanoic acid are removed by distillation.

4. Process according to any of Claims 1 to 3, **characterized in that** the reaction with formaldehyde in step c) is effected in the presence of a secondary amine and a mono-, di- or polycarboxylic acid.

5. Process according to one or more of Claims 1 to 4, **characterized in that** distillation is effected during the reaction with formaldehyde in step c).

6. Process according to one or more of Claims 1 to 5, **characterized in that** the secondary amine used is an alkylamine of the formula R¹-NH-R² where R¹ and R² are the same or different and are each alkyl radicals having 1 to 12 and preferably 3 to 5 carbon atoms.

7. Process according to one or more of Claims 1 to 6, **characterized in that** a monocarboxylic acid having 1 to 10 carbon atoms or a di- or polycarboxylic acid having 2 to 10 carbon atoms is used.

8. Process according to one or more of Claims 1 to 7, **characterized in that** the oxidation is effected in the presence of alkali metal or alkaline earth metal carboxylates.

9. Process according to Claim 8, **characterized in that** the alkali metal or alkaline earth metal carboxylates used are lithium carboxylate, potassium carboxylate, sodium carboxylate, calcium carboxylate or barium carboxylate.

10. Process according to one or more of Claims 1 to 9, **characterized in that** the oxidation is conducted in the liquid phase.

11. Process according to one or more of Claims 1 to 10, **characterized in that** the oxidizing agent used is oxygen or oxygen-containing gases.

12. Process according to one or more of Claims 1 to 11, **characterized in that** the reaction with formaldehyde is conducted continuously in a flow tube.

## Revendications

1. Procédé de fabrication d'acides hexanoïques isomères contenant de l'acide 2-méthylpentanoïque à partir des courants secondaires formés lors de la fabrication de pentanals, **caractérisé en ce que**
a) un mélange contenant des butènes linéaires est mis en réaction en présence de composés de métaux de transition du groupe VIII du tableau périodique des éléments avec du monoxyde de carbone et de l'hydrogène à température élevée et pression élevée pour former un mélange de pentanals ;
b) le mélange obtenu selon l'étape a) est séparé en un courant de matière enrichi en 2-méthylbutanal et 3-méthylbutanal et en un courant de matière enrichi en n-pentanal ;
c) le courant de matière enrichi en 2-méthylbutanal et 3-méthylbutanal selon l'étape b) est mis en réaction avec du formaldéhyde ;
d) le mélange réactionnel obtenu à l'étape c) est mis en réaction sélectivement en présence d'un catalyseur d'hydrogénation avec de l'hydrogène à température élevée et pression élevée pour former un mélange contenant du 2-méthylbutanal et des hexanals isomères ;
e) le mélange réactionnel obtenu selon l'étape d) est séparé en un courant de matière enrichi en 2-méthylbutanal et en un courant de matières enrichi en un mélange d'hexanals isomères ; et
f) le mélange d'hexanals isomères obtenu selon l'étape e) est oxydé en un mélange d'acides hexanoïques isomères contenant de l'acide 2-méthylpentanoïque.

2. Procédé de fabrication d'acides hexanoïques isomères contenant de l'acide 2-méthylpentanoïque à partir des courants secondaires formés lors de la fabrication de pentanals, **caractérisé en ce que**
a) un mélange contenant des butènes linéaires est mis en réaction en présence de composés de métaux de transition du groupe VIII du tableau périodique des éléments avec du monoxyde de carbone et de l'hydrogène à température élevée et pression élevée pour former un mélange de pentanals ;
b) le mélange obtenu selon l'étape a) est séparé en un courant de matière enrichi en 2-méthylbutanal et 3-méthylbutanal et en un courant de matière enrichi en n-pentanal ;
c) le courant de matière enrichi en 2-méthylbutanal et 3-méthylbutanal selon l'étape b) est mis en réaction avec du formaldéhyde ;
d) à partir du mélange réactionnel obtenu à l'étape c), un courant de matière enrichi en 2-méthylbutanal et un courant de matière appauvri en 2-méthylbutanal sont obtenus ;
e) le courant de matière appauvri en 2-méthylbutanal obtenu à l'étape d) est mis en réaction sélectivement en présence d'un catalyseur d'hydrogénation avec de l'hydrogène à température élevée et pression élevée pour former un mélange contenant des hexanals isomères ; et
f) le mélange réactionnel obtenu selon l'étape e) est oxydé et les acides hexanoïques isomères sont séparés par distillation.

3. Procédé de fabrication d'acides hexanoïques isomères contenant de l'acide 2-méthylpentanoïque à partir des courants secondaires formés lors de la fabrication de pentanals, **caractérisé en ce que**
a) un mélange contenant des butènes linéaires est mis en réaction en présence de composés de métaux de transition du groupe VIII du tableau périodique des éléments avec du monoxyde de carbone et de l'hydrogène à température élevée et pression élevée pour former un mélange de pentanals ;
b) le mélange obtenu selon l'étape a) est séparé en un courant de matière enrichi en 2-méthylbutanal et 3-méthylbutanal et en un courant de matière enrichi en n-pentanal ;
c) le courant de matière enrichi en 2-méthylbutanal et 3-méthylbutanal selon l'étape b) est mis en réaction avec du formaldéhyde ;
d) le mélange réactionnel obtenu à l'étape c) est mis en réaction sélectivement en présence d'un catalyseur d'hydrogénation avec de l'hydrogène à température élevée et pression élevée pour former un mélange contenant du 2-méthylbutanal et des hexanals isomères ; et
e) le mélange réactionnel obtenu selon l'étape d) est oxydé, puis l'acide 2-méthylbutyrique et un mélange d'acides hexanoïques isomères contenant de l'acide 2-méthylpentanoïque sont séparés par distillation.

4. Procédé selon l'une quelconque des revendications 1 à 3, **caractérisé en ce que** la réaction avec le formaldéhyde selon l'étape c) a lieu en présence d'une amine secondaire et d'un acide mono-, di- ou polycarboxylique.

5. Procédé selon une ou plusieurs des revendications 1 à 4, **caractérisé en ce qu'**une distillation a lieu pendant la réaction avec du formaldéhyde selon l'étape c).

6. Procédé selon une ou plusieurs des revendications 1 à 5, **caractérisé en ce qu'**une alkylamine de formule R¹-NH-R² est utilisée en tant qu'amine secondaire, R¹ et R² étant identiques ou différents et représentant des radicaux alkyle de 1 à 12, de préférence 3 à 5 atomes de carbone.

7. Procédé selon une ou plusieurs des revendications 1 à 6, **caractérisé en ce qu'**un acide monocarboxylique de 1 à 10 atomes de carbone ou un acide di- ou polycarboxylique de 2 à 10 atomes de carbone est utilisé.

8. Procédé selon une ou plusieurs des revendications 1 à 7, **caractérisé en ce que** l'oxydation a lieu en présence de carboxylates de métaux alcalins ou alcalino-terreux.

9. Procédé selon la revendication 8, **caractérisé en ce qu'**un carboxylate de lithium, de potassium, de sodium, de calcium ou de baryum est utilisé en tant que carboxylates de métaux alcalins ou alcalino-terreux.

10. Procédé selon une ou plusieurs des revendications 1 à 9, **caractérisé en ce que** l'oxydation est réalisée dans la phase liquide.

11. Procédé selon une ou plusieurs des revendications 1 à 10, **caractérisé en ce que** de l'oxygène ou des gaz contenant de l'oxygène sont utilisés en tant qu'oxydant.

12. Procédé selon une ou plusieurs des revendications 1 à 11, **caractérisé en ce que** la réaction avec du formaldéhyde est réalisée en continu dans un tube d'écoulement.
